# EUROPÄISCHE PATENTSCHRIFT

(11) **EP 1 005 492 B1**
(45) Veröffentlichungstag und Bekanntmachung des Hinweises auf die Patenterteilung: **11.04.2007**
(21) Anmeldenummer: 98901870.0
(22) Anmeldetag: 18.02.1998
(51) Int. Cl.: C07K 14/755, A61K 38/37

(54) **REINIGUNG VON VON WILLEBRAND-FAKTOR DURCH KATIONENAUSTAUSCHERCHROMATOGRAPHIE**
PURIFICATION OF VON WILLEBRAND FACTOR BY CATION EXCHANGER CHROMATOGRAPHY
PURIFICATION DU FACTEUR DE VON WILLEBRAND PAR CHROMATOGRAPHIE SUR ECHANGEUR DE CATIONS

(30) Priorität: 27.02.1997 AT 33797
(43) Veröffentlichungstag der Anmeldung: 07.06.2000
(73) Patentinhaber: Baxter Aktiengesellschaft, 1221 Wien (AT)
(72) Erfinder: FISCHER, Bernhard, A-1160 Wien (AT); SCHÖNBERGER, Öyvind, L., A-1180 Wien (AT); MITTERER, Artur, 2304 Orth/Donau (AT); FIEDLER, Christian, A-1100 Wien (AT); DORNER, Friedrich, A-1230 Wien (AT); EIBL, Johann, A-1180 Wien (AT)
(74) Vertreter: Weinzinger, Arnulf
(86) Internationale Anmeldenummer: PCT/AT1998/000034
(87) Internationale Veröffentlichungsnummer: WO 1998/038219

(56) Entgegenhaltungen:
- EP-A- 0 469 985
- EP-A- 0 600 480
- EP-A- 0 705 846
- WO-A-91/13093
- WO-A-93/15199

## Beschreibung

Die Erfindung betrifft ein Verfahren zur Gewinnung eines gereinigten von Willebrand-Faktor-(vWF) mittels Kationenaustauscherchromatographie.

Im Plasma zirkuliert der vWF in einer Konzentration von 5 - 10 mg/l zum Teil in Form eines nicht-kovalenten Komplexes mit Faktor VIII. Der vWF ist ein Glycoprotein, welches in verschiedenen Zellen des menschlichen Körpers gebildet und später in die Zirkulation freigesetzt wird. Dabei wird in den Zellen ausgehend von einer Polypeptidkette mit einem Molekulargewicht von ca. 225000 Da (vWF-Monomer) durch Ausbildung von mehreren Schwefelbrücken ein vWF-Dimer (primäres vWF-Dimer) mit einem Molekulargewicht von ca. 450000 Da synthetisiert. Aus den vWF-Dimeren werden wiederum durch Verknüpfung über Schwefelbrücken weitere Polymere des vWF mit immer höheren Molekulargewichten, bis ca. 20000000 Da hergestellt.

Ein wichtiges Kriterium zur Charakterisierung des vWF ist die Multimer-Struktur-Analyse durch Agarose Elektrophorese. Es wird vermutet, daß insbesondere den hochmolekularen vWF-Polymeren essentielle Bedeutung bei der Blutgerinnung zukommt. Die funktionelle Aktivität des vWF wird.üblicherweise durch die Ristocetin-Kofaktoraktivität (vWF:RistCoF) bestimmt. Als Merkmal für die Reinheit und spezifische Wirksamkeit des vWF wird das Verhältnis zwischen Aktivität und vWF-Antigenkonzentration (vWF:Ag) ermittelt. Die spezifische Aktivität eines Präparates nimmt mit zunehmendem vWF:RistCoF zu vWF:Ag-Verhältnis zu.

Der vWF erfüllt wichtige Funktionen im Rahmen der Hämostase. Er zirkuliert im Plasma zum Teil als Komplex mit Faktor VIII, der die Blutgerinnung als Cofaktor unterstützt. Faktor VIII wird durch die Komplexbildung mit vWF stabilisiert und vor proteolytischem Abbau geschützt. Eine weitere Aufgabe des vWF ist seine Beteiligung an der Thrombozytenaggregation, die einen wichtigen Beitrag zur primären Hämostase leistet. Dabei bindet der vWF an die Glycoproteine Ib und IIb/IIIa der Oberflächenrezeptoren der Thrombozyten und vernetzt damit die Thrombozyten zu einem Thrombozyten-Aggregat. Weiterhin bedeutend für die primäre Hämostase ist die Affinität des vWF zu Collagen, einem Bestandteil der extrazellulären Matrix, die in intakten Gefäßen keinen direkten Kontakt mit dem Blut hat, da sie durch einen Monolayer aus Endothelzellen vom Blutstrom abgeschirmt ist. Bei Verletzung von Blutgefäßen kommt es aber am Ort der Läsion durch lokale Ablösung der Endothelzellschicht zu einer direkten Exposition der Bestandteile der extrazellulären Matrix mit dem Blut. Durch seine Affinität zum Collagen ist der vWF in der Lage, das sich bildende Thrombozyten-Aggregat im geschädigten Gefäßbereich am exponierten Subendothel zu fixieren. Dadurch erfolgt ein erster, labiler Wundverschluß, der durch die nachfolgende Blutgerinnung verfestigt wird.

Das von Willebrand-Syndrom ist durch einen Mangel eines funktionellen von Willebrand-Faktors oder durch ein abnormes Spektrum in der Multimerzusammensetzung des von Willebrand Faktors charakterisiert. Bei Patienten mit von Willebrand-Syndrom kann es, trotz der.in der Regel normalen Syntheserate des Faktor VIII durch mangelnde Stabilisierung des Faktor VIII, zu einem Faktor VIII-Mangel kommen, der auf der stark verringerten Plasmahalbwertszeit dieses Gerinnungsfaktors beruht. Daher können Patienten mit von Willebrand-Syndrom ähnliche Symptome wie Hämophilie A-Patienten zeigen (phänotypische Hämophilie). Durch das Fehlen von funktionell aktivem vWF kann es bei Patienten mit von Willebrand-Syndrom auch zu Funktionsstörungen bei der ThrombozytenAggregation und -Adhäsion kommen, wodurch Defekte in der primären Hämostase auftreten können. Bedingt durch Störungen dieser vWF-vermittelten Vorgänge zeigen Patienten mit von Willebrand-Syndrom verlängerte Blutungszeiten.

Zur Behandlung des von Willebrand-Syndroms müssen daher vWF-Präparate verabreicht werden, die den Mangel an funktionell aktivem vWF ausgleichen. Dazu können Präparate eingesetzt werden, die auch zur Therapie der Hämophilie A verwendet werden, wie Kryopräzipitat oder daraus hergestellte Faktor VIII-Konzentrate, die Komplexe aus Faktor VIII und vWF enthalten. Allerdings werden zur Behandlung der Hämophilie A immer besser gereinigte Faktor VIII:C-Konzentrate eingesetzt, die den vWF nicht oder nur noch in Spuren enthalten. Da die Supplementierung von Patienten mit von Willebrand-Syndrom mit Faktor VIII nicht notwendig ist und die Faktor VIII-Applikation die Gefahr einer Induktion von inhibitorischen Faktor VIII-Antikörpern im Patienten birgt, wäre ein vWF-Präparat, das möglichst weitgehend frei von kontaminierendem Faktor VIII ist, zur Behandlung des von Willebrand-Syndroms besonders wünschenswert. Daher besteht ein Bedarf an reinen und virussicheren von Willebrand Faktor-Präparationen mit hoher spezifischer Aktivität.

In der Literatur sind verschiedene Verfahren zur Reinigung und Gewinnung von vWF beschrieben.

Die EP 0 503 991 beschreibt die.Reinigung von vWF aus humanem Kryopräzipitat durch drei aufeinanderfolgende chromatographische Schritte: 1. Anionenaustauscherchromatographie an TSK-DEAE-Fractogel und Elution des vWF durch 0,15 M NaCl; 2. Nochmalige Anionenaustauscherchromatographie an TSK-DEAE-Fractogel und Elution des vWF durch 0,17 M NaCl und 3. Affinitätschromatographie an Gelatin-Sepharose zur Abtrennung des kontaminierenden Fibrinogens. Dabei wurden Aminosäure- und Calciumionen-haltige Puffer verwendet.

Die WO 89/12065 beschreibt die Trennung von plasmatischem vWF von Faktor VIII und weiterer Proteine durch Bindung der Proteine an einen Anionenaustauscher und stufenweise Elution durch Erhöhung der Salzkonzentration. Die vWF-haltige Fraktion wurde ein zweites Mal über einen Anionenaustauscher chromatographiert und als Konzentrat gewonnen.

Die EP 0 469 985 offenbart die Reinigung von plasmatischem vWF aus Kryopräzipitat, wobei in einem ersten Schritt Faktor VIII selektiv bei einer Salzkonzentration von 250 mM an einen Anionenaustauscher gebunden wird, während vWF im Überstand verbleibt. Nach Erniedrigung der Salzkonzentration des vWF-haltigen Überstandes auf eine Salzkonzentration zwischen 100 mM und 150 mM wird vWF an einen zweiten Anionenaustauscher gebunden und bei pH 6,6 mit 300-350 mM NaCl eluiert. Dabei wird vWF mit einer Aktivität von mindestens 50 U/mg, der einen Anteil von Faktor VIII von < 2% enthält, gewonnen.
Die DE 39 04 354 beschreibt die Gewinnung von plasmatischem vWF aus Kryopräzipitat und die Trennung von vWF vom Faktor VIII durch selektive Adsorption von Faktor VIII an einen Anionenaustauscher, während vWF in Lösung bleibt. Dabei wird eine Lösung enthaltend 160 U/ml vWF gewonnen.

Die US 5,006,642 beschreibt die Gewinnung von vWF aus einer gemäß der US 4,361,509 als Nebenprodukt anfallenden Lösung aus vWF und chaotropem Agens durch Dialyse gegen einen geeigneten Puffer oder ein Entsalzen der Lösung durch einen weiteren chromatographischen Schritt.

Die EP 0 383 234 beschreibt die Herstellung eines vWF-Konzentrates mittels Anionenaustauscherchromatographie, wobei ein in einer Lösung enthaltener Faktor VIII/vWF-Komplex durch Zugabe eines calcium- und aminosäurehaltigen Puffers dissoziiert und ein vWF-Konzentrat gewonnen wird.

Die WO 96/10584 beschreibt ein Verfahren zur Gewinnung von hochreinem rekombinantem vWF mittels kombinierter Anionenaustausch/Heparin-Affinitätschromatographie und die EP 0 705 846 die Trennung von hoch- und niedermolekularen Fraktionen von rekombinantem vWF mittels Heparin-Affinitätschromatographie.

Die WO93/15199 beschreibt die Verwendung von Albumin zur Herstellung von Albumin-Fusionsproteine als Träger für diverse Proteine. Als optionelle Komponente eines solchen Fusionsproteins werden kleine Fragmente des vWF zur Verhinderung der Anhaftung an vWF und Plättchen genannt.

Um eine gereinigte vWF-Präparation mit hoher spezifischer Aktivität zu gewinnen, war es bisher notwendig, mehrere chromatographische Schritte zu kombinieren. Insbesondere die Herstellung von Präparaten, die insbesondere hochmolekulare vWF-Multimere enthielten, war bisher nur über eine Heparin-Affinitätschromatographie möglich. Heparin ist jedoch ein relativ kostspieliges Chromatographiematerial.

Aufgabe der vorliegenden Erfindung ist es, ein für die großtechnische Anwendung im industriellen Maßstab geeignetes Verfahren zur Gewinnung von gereinigtem vWF mit verbesserter spezifischer Aktivität zur Verfügung zu stellen. Das Verfahren sollte sowohl für die Reinigung von rekombinantem als auch plasmatischem vWF einsetzbar sein.

Erfindungsgemäß wird die Aufgabe dadurch gelöst, daß ein Verfahren zur Gewinnung von vWF zur Verfügung gestellt wird, bei dem vWF bei einer (niedrigen) Salzkonzentration von ≤ 250 mM an einen Kationenaustauscher gebunden wird und durch stufenweise, fraktionierte Elution bei einer Salzkonzentration von > 300 mM vWF, bestehend insbesondere aus hochmolekularen vWF-Multimeren mit hoher spezifischer Aktivität, gewonnen wird. Die Gewinnung und Anreicherung von vWF mit verbesserter Aktivität und Stabilität erfolgt insbesondere dadurch, daß durch stufenweise Erhöhung der Salzkonzentration erst Fraktionen enthaltend niedermolekulare vWF-Multimere, inaktive Abbauprodukte und unspezifische Begleitproteine bei einer mittleren Salzkonzentration abgetrennnt werden und Fraktionen, enthaltend hochmolekulare vWF-Multimere mit hoher spezifischer Aktivität, bei einer höheren Salzkonzentration gewonnen werden.

vWF wird üblicherweise aufgrund seines sauren isoelektrischen Punktes (IEP =5,5 bis 6) und seiner daraus resultierenden negativen Netto-Ladung im schwach sauren bis basischen Milieu über positiv geladene Anionenaustauscher aufgereinigt. Es war daher aufgrund von den bisher beschriebenen Verfahren zur Reinigung von vWF mittels positiv geladener Anionenaustauscher nicht zu erwarten, daß vWF bei einem pH-Wert, der oberhalb des IEP des vWF liegt und niedriger Salzkonzentration an eine negativ geladene Gelmatrix eines KatIonenaustauschers bindet und von dieser durch Erhöhung der Salzkonzentration selektiv eluierbar ist. Es war ebenfalls nicht zu erwarten, daß durch stufenweise Elution bei einer Salzkonzentration > 300 mM vWF, bestehend insbesondere aus hochmolekularen vWF-Multimeren, erhalten wird.

Im Rahmen der vorliegenden Erfindung wurde festgestellt, daß mit dem erfindungsgemäßen Verfahren ausgehend von einem unreinen biologischen Material gereinigte Fraktionen erhalten werden, die im wesentlichen frei von kontaminierenden Nukleinsäuren sind. - Dadurch werden durch das Verfahren neben den unspezifischen Begleitproteinen auch Nukleinsäuren aus Proteinpräparationen entfernt. Dieser Effekt kann mit herkömmlichen Verfahren mittels Anionenaustauscher nicht gezeigt werden, da Nukleinsäuren aufgrund ihrer negativen Ladung an den Anionenaustauscher binden, sich durch Erhöhung der Salzkonzentration wieder vom Anionenaustauscher ablösen und ins Eluat gelangen.

Bei der Reinigung des vWF ist insbesondere zu beachten, daß, bedingt durch die Größe des vWF von 500 000 bis mehrere Millionen, nur solche Trägermaterialien gute Reinigungen und Ausbeuten liefern, die das vWF-Molekül in der Diffusion und Verteilung in den verwendeten Trägermaterialien nicht behindern. Bei der Durchführung des erfindungsgemäßen Verfahren zur Reinigung von vWF mit hoher spezifischer Aktivität mittels Kationenaustauscher wird nicht nur eine Gelmatrix verwendet, die eine hohe Beladungskapazität besitzt, robust in der Handhabung ist und ein scharfes Elutionsprofil zeigt, sondern die sich auch im industriellen Maßstab ökonomisch einsetzen läßt. Damit wird das erfindungsgemäße Verfahren insbesondere für die Gewinnung von reinem vWF im großtechnischen Ansatz interessant.

Zur Durchführung des Verfahrens kann jeder bekannte Kationenaustauscher eingesetzt werden, wobei Kationenaustauscher mit einem Sulfopropyl- oder Carboxymethyl-Gruppen konjugierten Träger bevorzugt sind. Als gut geeignet haben sich zum Beispiel SP-Sepharose^{®} Fast Flow und CM-Sepharose^{®} Fast Flow (Pharmacia), Fractogel^{®} EMD-SO3 und Fractogel^{®} EMD COOH (Merck), Poros^{®} 10 SP und Poros^{®} 10 S (Perseptive Biosystems) und Toyopearl^{™} SP 550 C und Toyopearl^{™} CM-650 (M) (TosoHaas) erwiesen.

Als besonders günstig für die Gewinnung von gereinigtem vWF hat sich ein großporiges Gel mit Tentakelstruktur vom Typ Fractogel^{®}. EMD-S03 und Fractogel^{®} EMD COOH (Merck) erwiesen.

Die Adsorption des vWF an den Kationenaustauscher erfolgt vorzugsweise bei einer Salzkonzentration im Puffer von s 250 mM. Bevorzugte Adsoprtionspuffer weisen daher eine Salzkonzentration von 50 bis 250 mM, insbesondere im Bereich von 150 mM - 250 mM (z.B. 150 mM) auf. Durch stufenweise Erhöhung der Salzkonzentration im Puffer kann selektiv vWF bestehend im wesentlichen aus hochmolekularen vWF-Multimeren bei einer Salzkonzentration von > 300 mM eluiert werden. Niedermolekulare vWF-Multimere und proteolytische vWF-Abbauprodukte, die in der vWF-haltigen Lösung enthalten sind und die eine geringe spezifische Aktivität in bezug auf die vWF-Aktivität, insbesondere auf die Ristocetin-Kofaktor-Aktivität, die Kollagenbindungsaktivität und die spezifische Plättchenagglutinationsaktivität aufweisen, werden bei einer Salzkonzentration zwischen > 250 mM und ≤ 300 mM, vorzugsweise bei 300 mM vom Kationenaustauscher eluiert und abgetrennt.

Die Adsorption und Desorption des vWF kann in einem Puffer, enthaltend als Salz ein ein- oder zweiwertiges Metallion, erfolgen, wobei als Salz vorzugsweise NaCl verwendet wird.

Im erfindungsgemäßen Verfahren wird als Puffersystem zur Elution der an den.Kationenaustauscher gebundenen Proteine vorzugsweise eine Pufferlösung bestehend aus Puffersubstanzen, insbesondere Glycin, Phosphatpuffer oder Citratpuffer, und Salz verwendet. Der eingesetzte Puffer enthält dabei vorzugsweise keine Ca-Ionen.

Der Elutionspuffer kann einen pH-Wert im pH-Bereich zwischen 5,0 bis 8,5, vorzugsweise zwischen 6,0 und 8,0 aufweisen.

Das erfindungsgemäße Verfahren kann als Batch-Verfahren oder als Säulenchromatographie durchgeführt werden.

Die optimalen Parameter wie Salzkonzentration, pH-Wert und Temperatur zur Durchführung des erfindungsgemäßen Verfahrens sind jedoch jeweils abhängig vom verwendeten Kationenaustauschermaterial. Es liegt jedoch im allgemeinen Wissen eines Fachmannes, die im Rahmen der vorliegenden Erfindung offenbarten Bedingungen zur Durchführung des Verfahrens, für den jeweilig eingesetzen Kationenaustauschertyp zu optimieren.

Durch das erfindungsgemäße Verfahren wird insbesondere ein vWF gewonnen und angereichert, der insbesondere aus hochmolekularen vWF-Multimeren besteht. Niedermolekulare vWF-Multimere und vWF-Fragmente mit geringer spezifischer Plättchenagglutinations-Aktivität werden selektiv abgetrennt, so daß Fraktionen enthaltend insbesondere hochmolekulare vWF-Multimere mit hoher Aktivität und Spezifität erhalten werden.

Die gewonnene(n) vWF-Fraktion(en) ist (sind) im wesentlichen frei von niedermolekularen vWF-Multimeren, vWF-Fragmenten mit geringer spezifischer Aktivität, Faktor VIII-Komplex, Faktor VIII:C, unspezifischen Begleitproteinen und kontaminierenden Nukleinsäuren.

Als Ausgangsmaterial zur Gewinnung von gereinigtem vWF mittels dem erfindungsgemäßen Verfahren kann jede vWF-haltige Lösung eingesetzt werden. Ausgangsmaterialien sind insbesondere biologische Materialien wie Plasma, eine Plasmafraktion, Kryopräzipitat, oder ein Überstand oder ein Extrakt einer rekombinanten Zellkultur.

vWF-haltige Lösungen können jedoch auch angereicherte Proteinlösungen sein, die durch einen vorangegangenen Reinigungsschritt, etwa über Gelfiltration, Anionenaustauscherchromatographie, Affinitätschromatographie oder einer Kombination davon, vorgereinigt wurden. Durch diese vorgeschaltenen Verfahren wird insbesondere erreicht, daß vWF angereichert und unspezifische Begleitproteine, insbesondere Faktor VIII oder Faktor VIII-Komplex, selektiv abgetrennt werden.

Gemäß einer besonderen Ausführungsform des erfindungsgemäßen Verfahrens wird als Ausgangslösung eine über einen Anionenaustauscher angereicherte vWF-haltige Fraktion eingesetzt.

Mittels Anionenaustauscherchromatographie kann, abhängig von der Art der Durchführung der Anionenaustauscherchromatographie, vWF entweder als ungebundenes Material den Anionenaustauscher frei passieren oder an diesen adsorbieren. So wird vWF beispielsweise aus einer Plasmafraktion dadurch gewonnen und angereichert, daß sowohl vWF als auch Faktor VIII/vWF-Komplex bei geringer Ionenstärke und Salzkonzentration im schwach saurem Milieu an einen Anionenaustauscher binden. vWF wird vom Anionenaustauscher dann bei einer mittleren Salzkonzentration von 150 mM bis 250 mM selektiv eluiert, während Faktor VIII-Komplex und freier, nicht komplexierter Faktor VIII erst bei einer hohen Salzkonzentration von > 300 mM desorbieren. '

Eine angereicherte vWF-Fraktion kann auch dadurch erhalten werden, daß eine vWF-haltige Lösung bei einer mittleren Salzkonzentration zwischen 100 mM und 200 mM mit einem Anionenaustauscher behandelt wird, wobei Faktor VIII-Komplex an den Anionenaustauscher bindet, während vWF in Lösung bleibt. Gebundener Faktor VIII-Komplex kann anschließend vom Anionenaustauscher durch Erhöhung der Salzkonzentration gewonnen werden.

Gemäß einer besonderen Ausführungsform wird der in einer angereicherten Lösung mit einer Salzkonzentration von s 250 mM vorliegende vWF direkt aus dem Durchfluß oder Eluat bzw. als Überstand (beim Batch-Verfahren) gewonnen und gegebenenfalls, ohne Änderung der Ionenstärke oder Salzkonzentration an den Kationenaustauscher gebunden. Die Salzkonzentration kann jedoch, falls erforderlich, auch durch Verdünnung erniedrigt werden.

Diese Ausführungsform hat den besonderen Vorteil, daß eine einfache Kombination von Anionen-/Kationenaustauscherchromatographie möglich ist, ohne aufwendige Umpufferung, Dialyse o.ä. der angereicherten Proteine.

Damit kann durch einen ersten Chromatographieschritt eine angereicherte vWF-haltige Fraktion erhalten werden und durch anschließende Kationenaustauscherchromatographie eine Reinigung und Trennung von hochmolekularen und niedermolekularen vWF-Fraktionen erreicht werden. Es sind jedoch auch.andere Kombinationen, wie etwa Affinitäts-/Kationenaustauscherchromatographie, Anionenaustauscher-/Affinitäts-/Kationenaustauscherchromatographie möglich, um eine weitere Anreicherung und selektive Gewinnung von vWF mit hoher spezifischer Aktivität zu erreichen.

Mit dem oben beschriebenen erfindungsgemäßen Verfahren wird vWF mit hoher spezifischer Aktivität aus einem unreinen vWF-haltigen Material mindestens 80fach angereichert.

Da prinzipiell jedes biologische Material mit infektiösen Keimen kontaminiert sein kann, wird zur Herstellung eines virussicheren Präparates die gewonnene vWF-haltige Fraktion zur Inaktivierung bzw. Abreicherung von Viren behandelt. Dazu können alle aus dem Stand der Technik bekannten Verfahren wie chemisch/physikalische Methoden, Inaktivierung durch Kombination einer photoaktiven Substanz und Licht oder Abreicherung durch Filtration eingesetzt werden. Zur Inaktivierung von Viren eignet sich insbesondere eine Hitzebehandlung in Lösung bzw. in festem Zustand, welche sowohl lipidumhüllte als auch nicht-lipidumhüllte Viren verläßlich inaktivieren kann. Die Virusabreicherung erfolgt vorzugsweise durch eine Filtration über Nanofilter.

Gemäß einem weiteren Aspekt stellt die vorliegende Erfindung eine Präparation, enthaltend gereinigten vWF mit hoher spezifischer Aktivität, bestehend insbesondere aus hochmolekularen vWF-Multimeren, erhältlich aus einer vWF-haltigen Lösung durch Kationenaustauscherchromatographie nach der vorliegenden Erfindung, zur Verfügung. vWF mit hoher spezifischer Aktivität wird ausgehend von einem Ausgangsmaterial, enthaltend u.a.vWF mit geringer Reinheit und niedriger spezifischer Aktivität, angereichert und Begleitproteine, insbesondere Faktor VIII bzw. Faktor VIII-Komplex, enthaltend niedermolekulare vWF-Multimere, selektiv abgetrennt. Dadurch wird insbesondere eine Präparation, enthaltend gereinigten vWF, der insbesondere aus hochmolekularen vWF-Multimere besteht und im wesentlichen keine niedermolekularen vWF-Multimere und vWF-Abbauprodukte enthält, gewonnen.

Die erfindungsgemäße Präparation weist insbesondere eine spezifische Plättchenagglutinations-Aktivität von vWF von mindestens 65 U/mg Protein und eine spezifische Collagen-Bindungsaktivität von mindestens 65 U/mg Protein auf. Ebenso zeichnet sich die Präparation dadurch aus, daß sie im wesentlichen frei ist von Faktor VIII und einen Faktor VIII-Gehalt von < 0,1% in bezug auf das Verhältnis von Aktivität vWF zu Faktor VIII aufweist.

Ein weiteres Kriterium für die Reinheit und geringe Infektiösität eines Produktes ist auch die Abwesenheit von kontaminierenden Nukleinsäuren. Die erfindungsgemäße Präparation ist daher im wesentlichen frei von Nukleinsäuren. "Im wesentlichen" bedeutet hier, daß der Gehalt an Nukleinsäure s 0,7 bezogen auf das Verhältnis 260/280 nm ist. Die Nukleinsäure kann jedoch auch gemäß einem Verfahren, wie es beispielsweise in der EP 0 714 987 und der EP 0 714 988 beschrieben wird, quantifiziert werden.

Bei Gewinnung und Herstellung der erfindungsgemäßen Präparation mit plasmatischem vWF, aber auch mit rekombinantem vWF als Ausgangsmaterial wird, wie oben beschrieben, zur Entfernung von infektiösen Partikeln gegebenenfalls ein Virusabreicherungs/oder Inaktivierungsverfahren durchgeführt, wobei eine Virusinaktivierung und/oder Virusabreicherung prinzipiell vor oder nach jedem Reinigungsschritt ausgehend vom Ausgangsmaterial bis zur hergestellten pharmazeutischen Zubereitung erfolgen kann. Damit ist die erfindungsgemäße Präparation in jedem Fall virussicher.

Gemäß einer bevorzugten Ausführungsform liegt die erfindungsgemäße Präparation in einer lagerstabilen Form vor. Die Präparation, enthaltend gereinigten vWF mit hoher spezifischer Aktivität, kann als fertige Lösung, Lyophilisat oder tiefgefroren bereitgestellt werden. Aufgrund ihrer Reinheit ist die Präparation besonders stabil. So hat sich gezeigt, daß die erfindungsgemäße Präparation bei -20°C für mindestens 6 Monate, bei 4°C in Lösung für mindestens 4 Wochen und als Lyophilisat für mindestens 1 Jahr stabil ist. Es zeigte sich, daß innerhalb des jeweiligen Zeitraumes die vWF-Aktivität um maximal 10% reduziert wird und auch das Multimermuster der vWF-Multimere keine wesentliche Änderung zeigt.

Die Formulierung der erfindungsgemäßen Präparation kann in an sich bekannter und üblicher Weise erfolgen. Der gereinigte vWF, enthalten in der erfindungsgemäßen Präparation, wird mit einem Puffer enthaltend Salze, wie NaCl, Trinatrium-citratdihydrat und/oder CaCl₂, und Aminosäuren, wie Glycin und Lysin, bei einem pH im Bereich von 6 bis 8 gemischt und als pharmazeutisches Präparat formuliert.

Die Präparation kann zur Herstellung eines Arzneimittels zur Behandlung von Patienten mit phänotypischer Hämophilie und vWD verwendet werden.

Die Erfindung wird anhand der nachfolgenden Beispiele und der Zeichnungsfiguren näher erläutert, wobei sie jedoch nicht auf diese Ausführungsbeispiele beschränkt ist.

Es zeigen:
- Figur 1:: Multimer-Analyse von rvWF vor und nach Reinigung mittels Kationenaustauscherchromatographie
- Figur 2:: Multimer-Analyse von vWF aus Kryopräzipitat vor und nach Reinigung mittels kombinierter Anionen-/Kationenaustauscherchromatographie unter Bedingungen, unter denen vWF an den Anionenaustauscher bindet (Beispiel 2 A)
- Figur 3:: Multimer-Analyse von vWF aus Kryopräzipitat vor und nach Reinigung mittels kombinierter Anionen-/Kationenaustauscherchromatographie unter Bedingungen, unter denen vWF nicht an den Anionenaustauscher bindet (Beispiel 2 B)

Beispiel 1 beschreibt die Reinigung von rvWF aus Kulturüberständen rekombinanter Zellen mittels Kationenaustauscherchromatographie; Beispiel 2 beschreibt die Reinigung von plasmatischem vWF mittels Kationenaustauscherchromatographie und vorgeschaltener Anionenaustauscherchromatographie; Beispiel 3 beschreibt die Reinigung von rekombinantem vWF mittels kombinierter Anionenaustauscher-/Immunaffinitäts- und Kationenaustauscher-Chromatographie.

### Beispiel 1 :

### Reinigung von vWF aus Kulturüberständen rekombinanter Zellen mittels Kationenaustauscherchromatographie

vWF wurde in rekombinanten CHO-Zellen in einem üblichen Kulturmedium produziert. Nach der Fermentation der transformierten Zellen wurde das Kulturmedium abgenommen und Zellen und Zellbruchstücke durch Zentrifugation entfernt. Anschließend wurde die Lösung zur Entfernung von niedermolekularen Bestandteilen, wie Membranbruchstücke, durch Filter mit Porengröße von 0,4 µm geklärt.

Eine Chromatographiesäule (50 ml) wurde mit einem Kationenaustauscher (Fractogel^{®} EMD-SO3) gefüllt und mit Puffer (30 mM Glycin-NaC1-Puffer) gespült. Anschließend wurde die Kationenaustauschersäule mit dem zellfreien Kulturüberstand beladen, wobei solche Proteine, die nicht an den Austauscher binden, im Durchfluß erhalten wurden (Fraktion 1). Gebundene unspezifische Begleitproteine wurden durch Spülen der Säule mit Puffer, enthaltend 0,3 M NaCl, entfernt (Fraktion 2). Anschließend wurde gebundener vWF vom Austauscher mit Puffer, enthaltend 0,5 M NaC1, desorbiert und im Eluat erhalten (Fraktion 3).

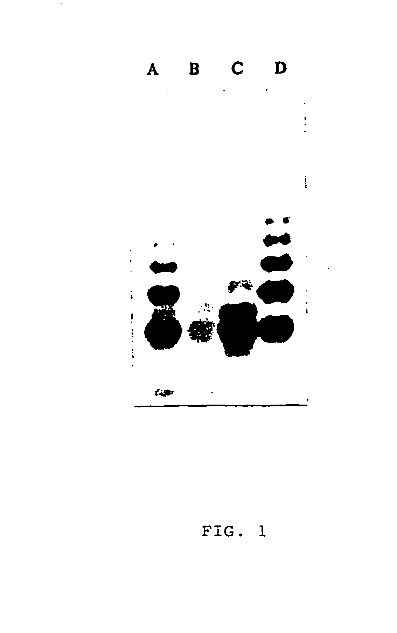

Alle Fraktionen wurden auf ihren Proteingehalt, vWF-Antigengehalt (vWF:Ag), vWF-Aktivität (Ristocetin-Kofaktor-Aktivität, vWF:RistCoF), untersucht und einer vWF-Multimer-Analyse unterzogen. Die Proteinkonzentration wurde mittels der Bradford-Methode (M. Bradford (1976), Anal. Biochem., 72: 248-254) bestimmt. Der Gehalt an vWF wurde mittels eines handelsüblichen ELISA-Systems (ASSERACHROM^{®}vWF, Boehringer Mannheim) bestimmt. Die Ristocetin-CoFaktor-Aktivität wurde mittels eines üblichen Testsystems (v-Willebrand-Reagenz^{®}, Behringwerke) ermittelt. Die Ergebnisse der Kationenaustauscherchromatographie sind in der Tabelle 1 zusammengefaßt. Fig. 1 zeigt die vWF-Multimer-Analyse vor und nach Reinigung über den Kationenaustauscher.

Aus der Tabelle 1 ist ersichtlich, daß der gesamte im Ausgangsmaterial vorhandene vWF mit Ristocetin-CoFaktor-Aktivität durch den Kationenaustauscher gebunden wird. Durch Spülen der Kationenaustauschersäule mit einem Puffer, enthaltend 0,3 M NaCl (Fraktion 2), wird vWF ohne meßbare Aktivität entfernt. Durch Elution mit 0,5 M NaCl (Fraktion 3) wird vWF mit nahezu aller Ristocetin-CoFaktor-Aktivität erhalten. Außerdem wird eine hohe Abreicherung von DNA des Kulturüberstandes erhalten: das Absorptionsverhältnis 260 nm / 280 nm fällt von 1,2 auf 0,7. Mit diesem einen chromatographischen Schritt ergibt sich ein Reinigungsfaktor von 10.

Figur 1 zeigt die Multimer-Analyse von vWF vor und nach Reinigung mittels Kationenaustauscherchromatographie. In Fig. 1, Spur A, ist ungereinigter rvWF, in Spur B vWF-Multimere der Fraktion ₁ im Durchlauf; in Spur C vWF-Multimere der Fraktion 2 (0,3 M NaCI-Eluat) und in Spur D die der Fraktion 3 (0,5 M NaC1-Eluat) gezeigt. Aus Figur 1 ist ersichtlich, daß durch die Kationenaustauscherchromatographie und selektive Elution ein vWF, enthaltend insbesondere hochmolekulare Multimerstrukturen erhalten wird. Niedermolekulare vWF-Multimere bzw. vWF-Abbauprodukte werden entweder nicht an den Kationen-Austauscher gebunden (Fraktion 1) oder durch Elution mit 0,3 M NaC1 selektiv abgetrennt (Fraktion 2).

**Tabelle 1 Reinigung von rekombinantem vWF (rvWF) mittels Kationenaustauscherchromatographie**

| **Probe** | **vWF:RistCoF (U/ml)** | **vWF:Ag (µg/ml)** | **UV-Absorptionsverhältnis 260 nm / 280 nm** |
|---|---|---|---|
| Ungereinigter vWF | 0,338 | 120,36 | 1,2 |
| Fraktion 1 (nicht gebunden) | 0 | 2,8 | 1,5 |
| Fraktion 2 (Eluat 0,3 M NaCl) | 0 | 10,7 | 0,7 |
| Fraktion 3 (Eluat 0,5 M NaCl) | 0,450 | 48,9 | 0,7 |

### Beispiel 2 :

### Reinigung von plasmatischem vWF über Kationenaustauscher mit vorgeschaltener Reinigung über Anionenaustauscher

### A. Anionenaustauscherchromatographie unter Bedingungen, unter denen vWF an den Anionenaustauscher bindet und selektive Elution von vWF

Kryopräzipitat aus humanem Plasma wurde in einem Puffer aus 7 mM Tris, 100 mM Na-Acetat, 100 mM Lysin bei pH 6,7 aufgelöst. Zur Vorbehandlung wurde AL(OH)₃ eingerührt. Anschließend wurde der Niederschlag durch Zentrifugation abgetrennt.

Auf diese Weise vorbehandeltes Kryopräzipitat wurde auf eine Anionenaustauschersäule Fractogel^{®} EMD-TMAE aufgetragen. Schwach gebundene Proteine wurde durch Spülen der Säule mit einem 160 mM NaCl-haltigen Puffer entfernt. Durch Elution mit 250 mM NaCl im Puffer wurde vorrangig vWF vom Austauscher eluiert (Fraktion 1). Durch Elution mit 400 mM NaCl wurde anschließend FVIII-Komplex eluiert (Fraktion 2). Ausgehend vom Kryopräzipitat enthielt die Fraktion 1 68% der gesamten vWF-Aktivität, jedoch nur 10 % der gesamten FVIII-Aktivität. Restliche vWF-Aktivität und 80 % der FVIII-Aktivität sind in der Fraktion 2 enthalten.

**Tabelle 2 Anreicherung von vWF mittels Anionenaustauscherchromatographie**

| **Probe** | **vWF:Rist CoF-Aktivität (U/ml)** | **FVIII:C Aktivität (U/ml)** |
|---|---|---|
| Kryopräzipitat | 13,5 | 13,6 |
| Fraktion 1 (Eluat 250 mM NaCl) | 5,4 | 2,8 |
| Fraktion 2 (Eluat 400 mM NaCl) | 4,5 | 11,8 |

Die vWF-haltige Fraktion 1 wurde anschließend auf eine Kationenaustauschersäule Fractogel^{®} EMD-SO3 aufgetragen. Schwach gebundene Proteine wurden durch Spülen der Säule mit 100 mM NaCl entfernt. Anschließend wurde stufenweise mit 200 mM NaCl (Fraktion 1), 300 mM NaC1 (Fraktion 2) und 400 mM NaC1 (Fraktion 3) eluiert. Über 70 % der gesamten vWF-Aktivität wurde in der 400 mM NaC1-Fraktion gefunden. Es wurde keine FVIII:C-Aktivität gefunden. Die Ergebnisse sind in Tabelle 3 zusammengefaßt.

**Tabelle 3 Stufenweise Elution von vWF-Fraktionen vom vom Kationenaustauscher**

| **Probe** | **vWF:Rist -CoF-Aktivität (U/ml)** |
|---|---|
| vWF Fraktion 1 (Tab.2) | 5,4 |
| Fraktion 1 (Eluat 200 mM NaCl) | 0 |
| Fraktion 2 (Eluat 300 mM NaCl) | 0,2 |
| Fraktion 3 (Eluat 400 mM NaCl) | 3,75 |

Während die spezifische Aktivität des vWF im Kryopräzipitat 0,6 U/mg Protein betrug, beträgt diese im 400 mM NaCl-Eluat nach Kationenaustauscherchromatographie 65 U/ml. Die spezifische Collagen-Bindungsaktivität stieg von 0,7 U/mg Protein im Kryopräzipitat auf 65 U/mg Protein im 400 mM NaCl-Eluat nach Kationenaustauscherchromatographie. Ausgehend von Kryopräzipitat wurde die Reinheit des vWF 100-fach erhöht.

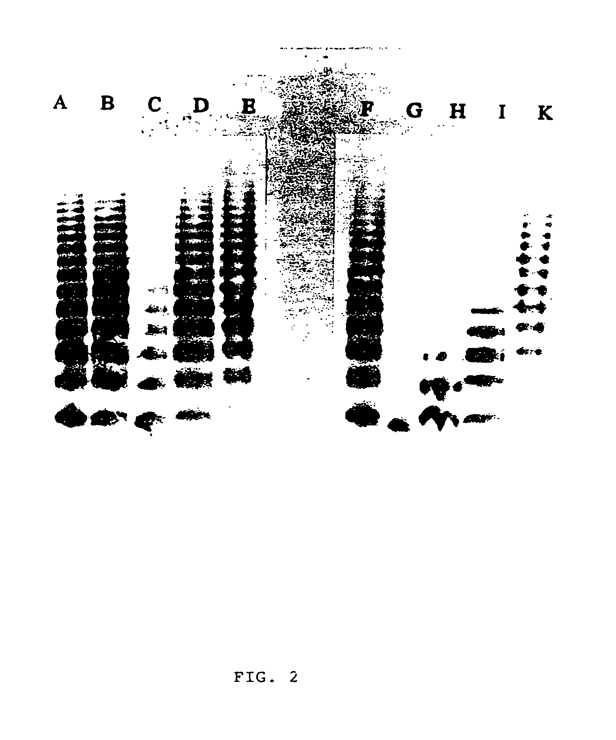

Figur 2 zeigt die vWF-Multimer-Analyse während der kombinierten Anionen-/Kationenaustauscherchromatoraphie. Die Spuren A bis E zeigen dabei die Reinigung über Anionenaustauscher und die Spuren F bis K die über Kationenaustauscher. Es zeigen Fig. 2, Spur A das vWF-Multimermuster von vWF im Kryopräzipitat, Spur B nach Filtration, Spur C im Durchlauf, Spur D das 250 mM NaCl-Eluat (Fraktion 1, Tabelle 2), Spur E das 400 mM NaC1-Eluat (Fraktion 2, Tabelle 2), Spur F das 250 mM NaC1-Eluat (Fraktion 1, Tabelle 2) vor der Kationenaustauscherchromatographie, Spur G den Durchlauf, Spur H das 200 mM NaC1-Eluat (Fraktion 1, Tabelle 3), Spur I das 300 mM NaC1-Eluat (Fraktion 2, Tabelle 3) und Spur K das 400 mM NaC1-Eluat (Fraktion 3, Tabelle 3). Aus Spur H und I ist erkennbar, daß durch Elution mit 200 mM NaCl bzw. 300 mM NaCl nur niedermolekulare vWF-Multimere vom Kationenaustauscher abgelöst werden. Elution des Kationenaustauschers mit 400 mM NaC1, Spur K, ergab vWF enthaltend insbesondere hochmolekulare vWF-Multimere.

### B. Anionenaustauscherchromatographie unter Bedingungen, unter denen vWF nicht an den Anionenaustauscher bindet und in Lösung verbleibt

Kryopräzipitat aus humanem Plasma wurde in einem Puffer aus 7 mM Tris, 100 mM Na-Acetat, 100 mM Lysin, 120 mM NaCl bei pH 6,7 aufgelöst. Zur Vorbehandlung wurde AL(OH)₃ eingerührt. Anschließend wurde der Niederschlag durch Zentrifugation abgetrennt.

Auf diese Weise vorbehandeltes Kryopräzipitat wurde auf eine Säule Fractogel^{®} EMD-TMAE aufgetragen. Nicht gebundene Proteine wurden durch Spülen der Säule mit Lösungspuffer erhalten (Fraktion 1). Diese Fraktion 1 enthielt 60 % der vWF-Aktivität und nur 10 % der FVIII-Aktivität. Durch Elution der Säule mit 400 mM NaCl (Fraktion 2) wurde anschließend FVIII-Komplex erhalten.

**Tabelle 4 Anreicherung von vWF mittels Anionenaustauscherchromatographie**

| **Probe** | **vWF:Rist CoF-Aktivität (U/ml)** | **FVIII:C Aktivität (U/ml)** |
|---|---|---|
| Kryopräzipitat | 12,5 | 12,2 |
| Fraktion 1 (vWF nicht gebunden) | 3,5 | 0,7 |
| Fraktion 2 Eluat 400 mM NaCl | 2,5 | 14,5 |

Die vWF-haltige Fraktion 1 wurde anschließend auf eine Kationenaustauschersäule (Fractogel^{®} EMD-S03) aufgetragen. Schwach gebundene Proteine wurden durch Spülen der Säule mit 100 mM NaCl entfernt. Anschließend wurde stufenweise mit 200 mM NaCl (Fraktion 1), 300 mM NaCl (Fraktion 2) und 400 mM NaCl (Fraktion 3) eluiert. Über 70 % der vWF-Aktivität wurden in der 400 mM NaCl-Fraktion gefunden. Es wurde kein Faktor VIII-Antigen und FVIII:C-Aktivität gefunden. Die Ergebnisse sind in Tabelle 5 zusammengefaßt.

**Tabelle 5 Stufenweise Elution von vWF-Fraktionen vom Kationenaustauscher**

| **Probe** | **vWF:Rist COF-Aktivität (U/ml)** |
|---|---|
| vWF (Fraktion 1, Tab. 4) | 3,5 |
| Fraktion 1 (Eluat 200 mM NaCl) | 0 |
| Fraktion 2 (Eluat 300 mM NaCl) | 0,2 |
| Fraktion 3 (Eluat 400 mM NaCl) | 3,75 |

Während die spezifische Aktivität des vWF im Kryopräzipitat 0,6 U/mg Protein betrug, beträgt diese im 400 mM NaC1-Eluat nach Kationenaustauscherchromatographie 47 U/mg. Die spezifische Collagen-Bindungsaktivität stieg von 0,7 U/mg Protein im Kryopräzipitat auf 51 U/mg Protein im 400 mM NaC1-Eluat nach Kationenaustauscherchromatographie. Ausgehend von Kryopräzipitat wurde die Reinheit des vWF 80-fach erhöht.

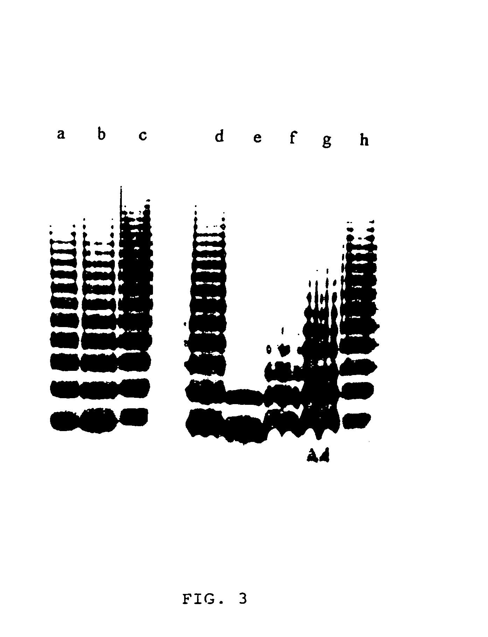

Figur 3 zeigt die vWF-Multimer-Analyse während der kombinierten Anionen-/Kationenaustauscherchromatographie. Die Spuren a bis c zeigen dabei Reinigung über Anionenaustauscher und die Spuren d bis h die über Kationenaustauscher. Es zeigen Fig. 3, Spur a das vWF-Multimermuster von vWF im Kryopräzipitat, Spur b im Durchlauf, Spur c das 400 mM NaCl-Eluat (Fraktion 2, Tabelle 4), Spur d den Durchlauf (Fraktion 1, Tabelle 4) vor der Kationenaustauscherchromatographie, Spur e den Durchlauf über Kationenaustauscher, Spur f das 200 mM NaCl-Eluat (Fraktion 1, Tabelle 5), Spur g das 300 mM NaC1-Eluat (Fraktion 2, Tabelle 5) und Spur h das 400 mM NaC1-Eluat (Fraktion 3, Tabelle 5). Die vWF-Multimer-struktur des vWF in den Fraktionen, die mit 200 mM NaCl (Spur f) bzw.,300 mM NaCl (Spur g), sowie 400 mM NaCl (Spur h) nach Kationenaustauscherchromatographie erhalten wurden, ist entsprechend dargestellt. Das 400 mM NaCl-Eluat (Spur h) zeigt ein hochmolekulares vWF-Multimermuster und enthält mehr als 70% der vWF-Aktivität (derzeit nach Ansicht der Anmelderin der beste Weg zur Ausführung der Erfindung).

### Beispiel 3 .

### Reinigung von rekombinantem vWF mittels kombinierter Anionenaustauscher-/Iamnunaffinitäts- und Kationenaustauscherchromatographie

vWF wurde durch rekombinante CHO-Zellen in einem üblichen Kulturmedium produziert. Nach der Fermentation der transformierten Zellen wurde das Kulturmedium abgenommen, Zellen und Zellbruchstücke wurden durch Zentrifugation entfernt. Anschließend wurde die Lösung zur Entfernung von niedermolekularen Bestandteilen, wie Membranbruchstücke, durch Filter mit Porengröße von 0,4 µm filtriert.

### Anionenaustauscher-Chromatographie

1000 ml zellfreier Kulturüberstand wurden mit einer Fließgeschwindigkeit von 0,7 cm/min über eine Säule (7,1 cm² x 8 cm, gefüllt mit 57 ml Anionenaustauscher Fractogel^{®} EMD-TMAE 650 M (Merck)) filtriert. Das Gel wurde zuvor mit 20 mM Tris-HCl- Puffer (pH 7,0) äquilibriert. Anschließend wurde die Säule mit 20 mM Tris-HCl-Puffer (pH 7,0), der 0,1 M NaCl enthielt, gewaschen. Begleitstoffe und vWF mit geringer RistCoF-Aktivität wurden durch Waschen der Säule mit 200 mM NaCl enthaltendem Puffer entfernt. Der rvWF wurde dann mit 280 mM NaCl in 20 mM Tris-HCl-Puffer (pH 7,0) vom Träger eluiert.

### Immunaffinitätschromatographie

Das 280 mM NaC1-Eluat des Anionenaustauscherschrittes wurde auf ein immobilisiertes Antikörperharz (Säulendimension: 19,6 cm² x 5,6 cm; Gelbettvolumen: 110 ml; Harzmatrix: Sepharose CL2B; Antikörper: Fab-Fragmente des murinen monoklonalen Antikörpers AvW8/2), das mit 20 mM Na-Acetat, 300 mM NaCl, (pH 7,0) äquilibriert worden war, mit einer Fließgeschwindigkeit von 0,255 cm/min geladen. Anschließend wurde mit 20 mM Na-Acetat, 300 mM NaCl (pH 7,0), dem 0,5% Tween 80 zugesetzt waren, gespült. r-vWF wurde mit 20 mM Glycin-Puffer, dem 10% Saccharose zugesetzt worden war, bei pH 8,0 eluiert. Nach 80% des Säulenvolumens wurde die Fließgeschwindigkeit um das rund 20-fache reduziert.

### Kationenaustauscher-Chromatographie

Eine Chromatographiesäule (7,1 cm² x 8 cm, gefüllt mit 57 ml Fractogel^{®} EMD-S03) wurde mit Puffer (30 mM Glycin-NaC1-Puffer; pH 5,0) gespült. Anschließend wurde das Eluat der Immunaffinitätschromatographie durch die Kationenaustauschersäule filtriert. Nach erneutem Waschen der Säule mit 30 mM Glycin-NaC1-Puffer wurden an den Kationenaustauscher gebundene Begleitstoffe und vWF mit geringer spezifischer Aktivität durch Spülen der Säule mit gepufferter 0,3 M NaC1-Lösung entfernt. Anschließend wurde vWF von der Austauschersäule durch Elution mit Puffer, enthaltend 0,5 M NaCl, in der Fraktion 3 erhalten.

Nach den einzelnen Chromatographie-Schritten wurde die Proteinkonzentration, der Gehalt an-rvWF-Antigen (vWF:Ag) und die Ristocetin-Kofaktor-Aktivität (vWF:RistCoF) ermittelt.

Es wurde gefunden, daß rvWF durch die Anionenaustauscherchromatographie um den Faktor 2,3 angereichert wird. In der darauffolgenden Immunaffinitätschromatographie erfolgte eine weitere Anreicherung um den Faktor 3,6. Durch die daran anschließende Kationenaustauscherchromatographie konnte der vWF nochmals um einen Faktor 5,2 angereichert werden. Außerdem konnten durch diesen Schritt die im Eluat der Immunaffinitätssäule vorhandenen Spuren an murinem Antikörper praktisch vollständig entfernt werden (<0,02 mg/1000 U vWF RistCoF-Aktivität). Durch anschließende Kationenaustauscherchromatographie erfolgte eine Abtrennung der niedermolekularen vWF-Multimere und Anreicherung von vWF mit hochmolekularen Multimerstrukturen, wodurch das Verhältnis von vWF:RistCoF- zu vWF:Ag-Aktivität um den Faktor 4 gesteigert wird.

Die Ergebnisse der Reinigung des rvWF in den unterschiedlichen Stufen dieses sequenziellen Reinigungsverfahrens sind in Tabelle 6 dargestellt.

**Tabelle 6 Reinigung von rekombinantem vWF mittels kombinierter Anionenaustauscher-/Immunaffinitäts- und Kationenaustauscher-Chromatographie**

| P**robe** | **Protein (mg/ml)** | **vWF:RistCoF (U/ml)** | **vWF:Ag (mg/ml)** | **vW:RistCoF/Protein (U/mg)** |
|---|---|---|---|---|
| Ungereinigter vWF | 0,626 | 1,013 | 0,116 | 1,62 |
| Eluat Anionenaustauscher | 0,684 | 2,588 | 0,322 | 3,78 |
| Eluat Immunaffinität | 0,198 | 2,700 | 0,348 | 13,64 |
| Eluat Kationenaustauscher | 0,046 | 2,250 | 0,074 | 59,20 |

## Patentansprüche

1. Verfahren zur Gewinnung von vWF, **dadurch gekennzeichnet, daß** vWF bei einer Salzkonzentration von ≤ 250 mM an einen Kationenaustauscher gebunden wird und durch fraktionierte Elution bei einer Salzkonzentration von > 300 mM vWF mit hoher spezifischer Aktivität, umfassend insbesondere hochmolekulare vWF-Multimere, gewonnen wird.

2. Verfahren nach Anspruch 1, **dadurch gekennzeichnet, daß** vWF-haltige Fraktionen, enthaltend hochmolekulare vWF-Multimere, in einem Puffer mit einem pH-Wert im Bereich zwischen 5,0 bis 8,5, vorzugsweise zwischen 6,0 und 8,0, eluiert werden.

3. Verfahren nach einem der Ansprüche 1 bis 2, **dadurch gekennzeichnet, daß** niedermolekulare vWF-Multimere, proteolytische vWF-Abbauprodukte mit geringer spezifischer Aktivität und unspezifische Begleitproteine bei einer Salzkonzentration zwischen > 250 mM und ≤ 300 mM, entfernt werden.

4. Verfahren nach einem der Ansprüche 1 bis 3, **dadurch gekennzeichnet, daß** der Kationenaustauscher ein Sulfopropyl- oder Carboxymethyl-Gruppen konjugierter Träger ist.

5. Verfahren nach einem der Ansprüche 1 bis 4, **dadurch gekennzeichnet, daß** vWF-haltige Fraktion(en), enthaltend insbesondere hochmolekulare vWF-Multimere, mit einer spezifischen Plättchenagglutinations-Aktivität von mindestens 65 U/mg Protein und einer spezifischen Collagen-Bindungsaktivität von mindestens 65 U/mg Protein gewonnen werden.

6. Verfahren nach einem der Ansprüche 1 bis 5, **dadurch gekennzeichnet, daß** die gewonnene(n) vWF-haltige Fraktion(en) im wesentlichen frei ist (sind) von niedermolekularen vWF-Multimeren, vWF-Fragmenten mit geringer spezifischer vWF-Aktivität und kontaminierenden Nukleinsäuren.

7. Verfahren nach einem der Ansprüche 1 bis 6, **dadurch gekennzeichnet, daß** vWF mit hoher spezifischer Aktivität aus Plasma, einer Plasmafraktion, Kryopräzipitat, dem Überstand oder Extrakt einer rekombinanten Zellkultur, oder einer angereicherten Proteinlösung gewonnen wird.

8. Verfahren nach Anspruch 7, **dadurch gekennzeichnet, daß** die angereicherte Proteinlösung durch einen vorangegangenen Reinigungsschritt erhalten wird.

9. Verfahren nach Anspruch 8, **dadurch gekennzeichnet, daß** die angereicherte Proteinlösung durch ein chromatographisches Verfahren, wie Anionenaustauscher- oder Affinitätschromatographie oder einer Kombination davon erhalten wird.

10. Verfahren nach einem der Ansprüche 1 bis 9, **dadurch gekennzeichnet, daß** die gewonnene(n) vWF-haltige(n) Eraktion(en) zur Inaktivierung bzw. Abreicherung von Viren behandelt wird (werden).

11. Präparation enthaltend gereinigten vWF, bestehend insbesondere aus hochmolekularen vWF-Multimeren, erhältlich aus einer vWF-haltigen Proteinlösung durch ein Verfahren nach einem der Ansprüche 1 bis 10.

12. Präparation nach Anspruch 11, **dadurch gekennzeichnet, daß** sie im wesentlichen keine niedermolekularen vWF-Multimere, inaktive vWF-Abbauprodukte und kontaminierende Nukleinsäuren enthält.

13. Präparation nach Anspruch 11 oder 12, **dadurch gekennzeichnet, daß** sie vWF mit einer spezifischen Plättchenagglutinationsaktivität von mindestens 65 U/mg Protein und einer spezifischen Collagen- Bindungsaktivität von mindestens 65 U/mg Protein enthält.

14. Präparation nach einem der Ansprüche 11 bis 13, **dadurch gekennzeichnet, daß** sie im wesentlichen frei ist von Faktor VIII und einen Faktor VIII-Gehalt von < 0,1% in bezug auf das Verhältnis von Aktivität vWF zu Faktor VIII:C aufweist.

15. Präparation nach einem der Ansprüche 11 bis 14, **dadurch gekennzeichnet, daß** sie virussicher ist.

16. Präparation nach einem der Ansprüche 11 bis 15, **dadurch gekennzeichnet, daß** sie in einer lagerstabilen Form vorliegt.

17. Präparation nach einem der Ansprüche 11 bis 16, **dadurch gekennzeichnet, daß** sie als pharmazeutisches Präparat formuliert ist.

18. Verwendung einer vWF-haltigen Präparation nach einem der Ansprüche 11 bis 17 zur Herstellung eines Arzneimittels zur Behandlung von Patienten mit phänotypischer Hämophilie und vWD.

## Claims

1. A method for recovering vWF, **characterised in that** vWF is bound to a cation exchanger at a salt concentration of ≤ 250 mM and **in that** vWF of a high specific activity, particularly comprising high-molecular vFW multimers, is recovered by fractionated elution at a salt concentration of > 300 mM.

2. The method according to claim 1, **characterised in that** vWF-containing fractions, containing high-molecular vWF multimers, are eluted in a buffer having a pH value ranging from 5.0 to 8.5, preferably from 6.0 to 8.0.

3. The method according to any one of claims 1 to 2, **characterised in that** low-molecular vWF multimers, proteolytic vWF degradation products having a low specific activity and unspecific accompanying proteins are removed at a salt concentration of between > 250 mM and ≤ 300 mM.

4. The method according to any one of claims 1 to 3, **characterised in that** the cation exchanger is a sulfopropyl or carboxymethyl group conjugated carrier.

5. The method according to any one of claims 1 to 4, **characterised in that** a vWF-containing fraction(s), particularly containing high-molecular vWF multimers, having a specific platelet agglutinating activity of at least 65 U/mg of protein and a specific collagen-binding activity of at least 65 U/mg of protein are recovered.

6. The method according to any one of claims 1 to 5, **characterised in that** the vWF-containing fraction(s) recovered is/are substantially free from low-molecular vWF multimers, from vWF-fragments having a low specific vWF activity and from contaminating nucleic acids.

7. The method according to any one of claims 1 to 6, **characterised in that** vWF having a high specific activity is recovered from plasma, a plasma fraction, cryoprecipitate, the supernatant or the extract of a recombinant cell culture or from an enriched protein solution.

8. The method according to claim 7, **characterised in that** the enriched protein solution is obtained by a preceding purification step.

9. The method according to claim 8, **characterised in that** the enriched protein solution is obtained by a chromatographic method, such as anion exchange chromatography or affinity chromatography, or a combination thereof.

10. The method according to any one of clams 1 to 9, **characterised in that** the recovered vWF-containing fraction(s) is/are treated for virus inactivation and virus depletion, respectively.

11. A preparation containing purified vWF, particularly consisting of high-molecular vWF multimers, obtainable from a vWF-containing protein solution by a method according to any one of claims 1 to 10.

12. The preparation according to claim 11, **characterised in that** said preparation is substantially free from low-molecular vWF multimers, inactive vWF degradation products and contaminating nucleic acids.

13. The preparation according to claim 11 or 12, **characterised in that** said preparation contains vWF having a specific platelet agglutinating activity of at least 65 U/mg of protein and a specific collagen-binding activity of at least 65 U/mg of protein.

14. The preparation according to any one of claims 11 to 13, **characterised in that** said preparation is substantially free from factor VIII and has a factor-VIII content of < 0.1 %, based on the ratio of activity of vWF to factor VIII:C.

15. The preparation according to any one of claims 11 to 14, **characterised in that** said preparation is virus-safe.

16. The preparation according to any one of claims 11 to 15, **characterised in that** said preparation is present in a storage-stable form.

17. The preparation according to any one of claims 11 to 16, **characterised in that** said preparation is formulated as pharmaceutical preparation.

18. Use of a vWF-containing preparation according to any one of claims 11 to 17 to produce a medicament for the treatment of patients suffering from phenotypic hemophilia and vWD.

## Revendications

1. Procédé d'obtention de vWF, **caractérisé en ce que**, à une concentration de sel de ≤ 250 mM, le vWF est fixé sur un échangeur de cations et, à une concentration de sel de > 300 mM, est obtenu par élution fractionnée avec une haute activité spécifique, comprenant en particulier des multimères du vWF.

2. Procédé selon la revendication 1, **caractérisé en ce que** les fractions contenant le vWF, qui contiennent des multimères de vWF de haut poids moléculaire, sont éluées dans un tampon avec un pH dans l'intervalle entre 5,0 et 8,5, de préférence entre 6,0 et 8,0.

3. Procédé selon l'une quelconque des revendications 1 à 2, **caractérisé en ce que** des multimères de vWF de bas poids moléculaire, des produits de dégradation protéolytique du vWF avec une faible activité spécifique et des protéines accompagnatrices inspécifiques sont éliminés à une concentration en sel de > 250 mM et ≤ 300 mM.

4. Procédé selon l'une quelconque des revendications 1 à 3, **caractérisé en ce que** l'échangeur de cations est un porteur conjugué de groupes sulfopropyles ou carboxyméthyles.

5. Procédé selon l'une quelconque des revendications 1 à 4, **caractérisé en ce qu'**une ou des fractions contenant le vWF, contenant en particulier des multimères de vWF de haut poids moléculaire, sont obtenues avec une activité spécifique d'agglutination de plaquettes d'au moins 65 U/mg de protéine et une activité spécifique de fixation du collagène d'au moins 65 U/mg.

6. Procédé selon l'une quelconque des revendications 1 à 5, **caractérisé en ce que** la ou les fractions de vWF obtenues sont exemptes de multimères de vWF de bas poids moléculaire, de fragments de vWF avec une faible activité de vWF, et d'acides nucléiques contaminateurs.

7. Procédé selon l'une quelconque des revendications 1 à 6, **caractérisé en ce que** du vWF avec une haute activité spécifique est obtenu à partir de plasma, d'une fraction de plasma, d'un cryoprécipité, du liquide surnageant ou d'un extrait d'une culture cellulaire recombinante, ou d'une solution de protéines enrichie.

8. Procédé selon la revendication 7, **caractérisé en ce que** la solution de protéines enrichie est obtenue par une étape de purification la précédant.

9. Procédé selon la revendication 8, **caractérisé en ce que** la solution de protéines enrichie est obtenue par un procédé chromatographique, tel une chromatographie sur échangeurs d'anions ou une chromatographie par affinités, ou par une combinaison des deux.

10. Procédé selon l'une quelconque des revendications 1 à 9, **caractérisé en ce que** la ou les fractions obtenues, contenant du vWF, sont traitées pour une inactivation, respectivement un appauvrissement des/en virus.

11. Préparation contenant du vWF purifié, consistant en particulier en multimères de vWF de haut poids moléculaire, pouvant être obtenue à partir d'une solution de protéines contenant du vWF par un procédé selon l'une quelconque des revendications 1 à 10.

12. Préparation selon la revendication 11, **caractérisée en ce qu'**elle ne contient essentiellement pas de multimères de vWF de bas poids moléculaire, de produits inactifs de décomposition du vWF, ni d'acides nucléiques contaminateurs.

13. Préparation selon la revendication 11 ou 12, **caractérisée en ce qu'**elle contient du vWF avec une activité spécifique d'agglutination de plaquettes d'au moins 65 U/mg de protéine et une activité spécifique de fixation du collagène d'au moins 65 U/mg.

14. Préparation selon l'une quelconque des revendications 11 à 13, **caractérisée en ce qu'**elle est essentiellement exempte de facteur VIII et qu'elle comporte une teneur en facteur VIII de < 0,1 % en référence au rapport de l'activité du vWF au facteur VIII: C.

15. Préparation selon l'une quelconque des revendications 11 à 14, **caractérisée en ce qu'**elle est virologiquement sûre.

16. Préparation selon l'une quelconque des revendications 11 à 15, **caractérisée en ce qu'**elle se présente sous une forme stable au stockage.

17. Préparation selon l'une quelconque des revendications 11 à 16, **caractérisée en ce qu'**elle est formulée comme préparation pharmaceutique.

18. Utilisation d'une préparation selon l'une quelconque des revendications 11 à 17 à la production d'un médicament pour le traitement de patients atteint d'hémophilie phénotypique.
